# EUROPÄISCHE PATENTSCHRIFT

(11) **EP 0 919 198 B1**
(45) Veröffentlichungstag und Bekanntmachung des Hinweises auf die Patenterteilung: **28.01.2004**
(21) Anmeldenummer: 98121293.9
(22) Anmeldetag: 09.11.1998
(51) Int. Cl.: A61B 17/68

(54) **Fixationselement zur Osteosynthese**
Osteosynthetic fixation element
Elément de fixation pour l'ostéosynthèse

(30) Priorität: 19.11.1997 DE 19751284
(43) Veröffentlichungstag der Anmeldung: 02.06.1999
(60) Teilanmeldung: 03018419.6
(73) Patentinhaber: Tutogen Medical GmbH, 91058 Erlangen-Tennenlohe (DE)
(72) Erfinder: Gotzen, Leo, Prof. Dr., 35041 Marburg (DE)
(74) Vertreter: Schmidt, Christian, Dipl.-Phys.

(56) Entgegenhaltungen:
- WO-A-97/25945
- WO-A-97/37603
- US-A- 6 007 539

## Beschreibung

Die vorliegende Erfindung betrifft ein Fixationselement zur Osteosynthese im menschlichen oder tierischen Körper. Derartige Fixationselemente sind grundsätzlich bekannt und dienen dazu, z.B. bei distalen Radiusfraktoren Knochenfragmente zu fixieren. Hierbei ist es im Rahmen der so genannten Bohrdrahtosteosynthese bekannt, distale Radiusfrakturen mit Kirschnerdrähten zu stabilisieren, was jedoch in mehrfacher Hinsicht nachteilig ist. Zum einen kann es nach dem Einsetzen dieser Metalldrähte zu Infekten kommen. Zum anderen können Metallallergien auftreten. Schließlich ist nach der Heilung der Fraktur bei Verwendung derartiger Fixationselemente stets eine zweite Operation erforderlich, in der die Metallelemente entfernt werden.

Ferner ist es bekannt, zur Fixation von Frakturen Fixationselemente aus Kunststoff zu verwenden, die im Körper resorbiert werden. Derartige Kunststoffelemente führen jedoch in einem hohen Prozentsatz zu Fremdkörperreaktionen und Osteolysen. Zudem nimmt ihre Biegefestigkeit in einem Zeitraum von etwa 2 - 3 Wochen sehr stark ab, so dass die für die Knochenheilung notwendige Stabilität nicht ausreichend gewährleistet ist.

Aus der WO 97/37603 ist ein Fixationselement bekannt, das aus Knochenmaterial besteht und als zylindrischer Stift ausgebildet ist.

Es ist das der Erfindung zugrunde liegende Problem (Aufgabe), ein Fixationselement der eingangs genannten Art zu schaffen, mit dem eine stabile und dauerhafte Fixierung von Knochenfragmenten erzielt werden kann und bei dem das Einbringen und Verankern im Knochenmaterial erleichtert ist.

Die Lösung dieser Aufgabe erfolgt durch die Merkmale des Anspruchs 1.

Erfindungsgemäß wird ein Fixationselement aus kortikalem Knochen eingesetzt, das an sich eine geringere Festigkeit als ein Kirschnerdraht aufweist. Jedoch hat sich herausgestellt, dass es ausreichend ist, ein schwächeres Material als Metall zur Fixation von Knochenfragmenten heranzuziehen, wobei sich bei Verwendung von Fixationsimplantaten aus kortikalem Knochenmaterial die Vorteile ergeben, dass Fremdkörperreaktionen entfallen, keine Metallallergien auftreten können und eine zweite Operation zur Entfernung des Fremdkörpers wegfallen kann. Hierdurch sind insgesamt die Kosten des Heilungsprozesses stark reduziert, wobei auch die mit einer zusätzlichen Narkose oder zweiten Operation verbundenen Gefahren entfallen.

Das erfindungsgemäße Fixationselement verankert sich in den Knochenfragmenten, wodurch die Osteosynthese stabiler als bei der Verwendung von resorbierbaren Kunststoffstiften wird. Zudem wird das kortikale Knochenmaterial in körpereigenes Knochenmaterial umgewandelt, so dass eine bestmögliche Heilung der Fraktur erzielt werden kann.

Erfindungsgemäß können kortikale Fixationselemente Verwendung finden, die aus allogenem oder xenogenem Knochenmaterial hergestellt sind.

Im Bereich des vorderen Endes des als Stift oder Nagel ausgebildeten Fixationselements ist ein Abschnitt mit verringertem Querschnitt vorgesehen. Hierdurch ergibt sich der große Vorteil, dass das Fixationselement in einer Bohrung an der Gegenkortikalis des Radiusknochens verankert werden kann, wobei der Hauptkörper des Fixationselementes nicht durch diese Bohrung durchrutschen kann, was zu einer sehr stabilen Verbindung führt.

Des Weiteren verläuft der Übergang zu dem Abschnitt mit verringertem Querschnitt konisch, wobei sich ein Winkel von etwa 45° zur Längsachse des Fixationselementes als besonders vorteilhaft herausgestellt hat, um eine feste Verankerung in der Gegenkortikalis zu erzielen.

Zusätzlich ist das vordere Ende des Fixationselementes konisch angespitzt.

Vorteilhafte Ausführungsformen der Erfindung sind in der Beschreibung, den Figuren sowie den Unteransprüchen beschrieben.

Der Abschnitt mit verringertem Querschnitt schließt sich vorzugsweise an das konisch angespitzte Ende des Fixationselementes an, wodurch das Fixationselement leicht in die vorgesehenen Bohrungen eingebracht werden kann.

Es ist vorteilhaft, wenn der Querschnitt des verjüngten Abschnittes etwa 80 bis 85 % des Querschnittes des übrigen Fixationselementes beträgt, vorzugsweise zwischen 3 und 5 mm².

Nachfolgend wird die folgende Erfindung rein beispielhaft anhand vorteilhafter Ausführungsformen und unter Bezugnahme auf die beigefügten Zeichnungen beschrieben. Es zeigen.

Der verjüngte Abschnitt kann sich über etwa 10 bis 20 % der Gesamtlänge des Fixationselementes erstrecken, vorzugsweise über etwa 10 mm. Eine solche Länge ist ausreichend, um eine gute und feste Verankerung in der Gegenkortikalis zu gewährleisten.

Nach einer weiteren Ausführungsform der Erfindung ist das Fixationselement als Schraube ausgebildet und weist vorzugsweise Ansatzflächen für einen Schraubenschlüssel auf. Derartige Schrauben als Fixationselemente sind zwar grundsätzlich bekannt, jedoch ergeben sich durch die Verwendung von kortikalem Knochenmaterial die eingangs erwähnten zusätzlichen Vorteile.

Bei dem schraubenartigen Fixationselement ist es besonders vorteilhaft, wenn der Übergang zwischen dem Schraubenkopf und dem Schraubenkörper konisch verläuft, wodurch ein Kragen gebildet wird, der sich auf dem zu fixierenden Knochenfragment abstützt. Der Kragen erlaubt es, den überstehenden Schraubenkopf abzusägen, falls dies notwendig ist.

Ein nicht beanspruchtes Setzwerkzeug für stiftförmige Fixationselemente weist zwei hülsenartige Führungen auf, die unter einem wählbaren spitzen Winkel zueinander fixierbar sind. Hierdurch lassen sich die erfindungsgemäßen Fixationselemente mit Hilfe eines Führungsdrahtes in verschiedenen Ebenen und unter verschiedenen Winkeln erleichtert einsetzen. Durch die zueinander verstellbaren hülsenartigen Führungen läßt sich der Winkelgrad zwischen dem Führungsdraht, der durch eine der Hülsen gesteckt ist, und dem einzusetzenden Fixationselement, das durch die andere Führung gehalten wird, leicht einstellen.
- Fig. 1: eine Seitenansicht eines als Stift ausgebildeten Fixationselementes;
- Fig. 2: eine Querschnittsansicht sowie eine Draufsicht eines schraubenartigen Fixationselementes; und
- Fig. 3: eine Seitenansicht eines Setzwerkzeuges.

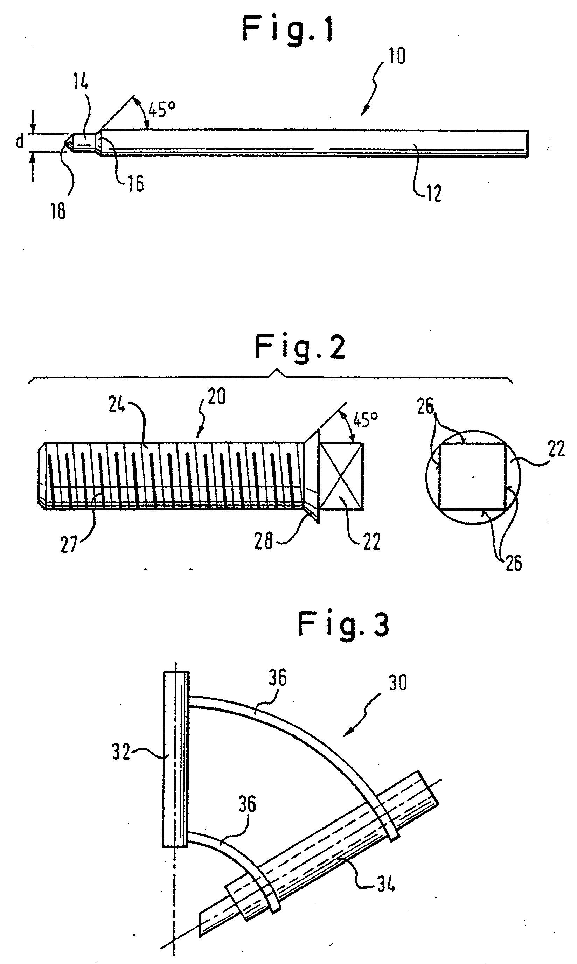

Die Fig. 2 und 3 betreffen Ausführungsformen, die nicht beansprucht sind.

Das in Fig. 1 dargestellte Fixationselement 10 ist stiftartig ausgebildet und dient zur Osteosynthese im menschlichen oder tierischen Körper. Das Fixationselement ist aus kortikalem Knochenmaterial hergestellt, das konserviert und sterilisiert ist. Der Fixationsstift 10 besitzt einen Hauptkörper 12 mit zylindrischem Querschnitt, an dessen vorderem Ende ein Abschnitt 14 mit verringertem Querschnitt vorgesehen ist. Der Übergang 16 zwischen dem Hauptkörper 12 und dem Abschnitt mit verringertem Querschnitt verläuft konisch und unter einem Winkel von etwa 45° zur Längsachse des Fixationsstiftes.

Die Spitze 18 des Fixationsstiftes 10 ist unter einem Winkel von 45° konisch angespitzt. Somit befindet sich der verjüngte Abschnitt 14 zwischen der konischen Spitze 18 und dem konischen Übergangsbereich 16.

Der Querschnitt des Abschnitts 14 beträgt etwa 80 bis 85 % des Querschnitts des Hauptkörpers 12. Im dargestellten Ausführungsbeispiel beträgt der Durchmesser d des verjüngten Abschnittes 14 2,5 mm, wohingegen der Durchmesser des Hauptkörpers 12 3 mm beträgt. Die Gesamtlänge des Fixationsstiftes 10 beträgt 10 mm, wobei die axiale Länge von der vorderen Spitze bis zum Ende des Übertragungsbereiches 16 10 mm beträgt.

Bei einer weiteren (nicht dargestellten) Ausführungsform beträgt der Durchmesser des Hauptkörpers 12 2,5 mm und der Durchmesser des verjüngten Abschnittes 2,0 mm. Bei beiden Ausführungsformen ist der Fixationsstift im Querschnitt kreisförmig ausgebildet.

Fig. 2 zeigt ein (nicht beanspruchtes) Fixationselement in Form einer Schraube 20, die einen Schraubenkopf 22 und einen Schraubenkörper 24 aufweist. An dem Schraubenkopf 22 sind Ansatzflächen 26 für einen Schraubenschlüssel vorgesehen. Der Schraubenkörper 24 besitzt ein Whitworth-Gewinde 27 und ist an seinem vorderen Ende angespitzt. Hierbei sind sowohl Schraubenkörper 24 wie auch Schraubenkopf 22 zylindrisch ausgebildet.

Der Übergang zwischen dem Schraubenkopf 22 und dem Schraubenkörper 24 verläuft bei der in Fig. 2 dargestellten Fixationsschraube 20 konisch unter einem Winkel von 45°, wodurch eine Abstützfläche in Form eines Kragens 28 geschaffen ist. Insbesondere wenn nach Einsetzen der Fixationsschraube 20 der überstehende Schraubenkopf 22 abgesägt wird, dient dieser Kragen 28 zur Abstützung auf das zu fixerende Knochenfragment.

Die Gesamtlänge der in Fig. 2 dargestellten Fixationsschraube 20 beträgt 24 mm, wobei die Länge des Schraubenkörpers 24 20 mm beträgt. Schraubenlänge und -durchmesser können jedoch variiert werden.

Fig. 3 zeigt ein Setzwerkzeug 30 für Fixationsstifte, das zwei hülsenartige Führungen 32 und 34 aufweist, die über gekrümmte Schienen 36 unter einem wählbaren spitzen Winkel zueinander fixierbar sind. Hierbei dient die Führung 32 zum Einsetzen eines Führungsdrahtes und die Führung 34 zum Einsetzen des Fixationselementes.

Zum Einsetzen des in Fig. 1 dargestellten Fixationsstiftes 10 wird nach einer Fragmentreposition ein entsprechendes Bohrloch angelegt, wobei das Bohrinstrument gleichzeitig als Führungsdraht für das Setzwerkzeug 30 dient, das auf diesen aufgesteckt wird. Durch eine Stichinzision wird das Setzwerkzeug bis an den Knochen herangeführt und fixiert. Für den Fixationsstift 10 wird durch die Führung 34 ein Bohrloch am Radiusknochen so angelegt, daß in der Gegenkortikalis eine Bohrung mit einem Durchmesser vorhanden ist, der dem verjüngten Ende 14 entspricht. Anschließend wird der Fixationsstift 10 in die Führung 34 des Setzwerkzeuges 30 eingelegt und mit einem Stößel und einem Hammer eingebracht. Anschließend kann das Setzwerkzeug 30 entfernt werden.

Zum Einbringen der in Fig. 2 dargestellten Fixationsschraube 20 wird nach Schaffen des üblichen operativen Zugangs und der Reposition der Fragmente ein Zugloch gebohrt, das mit einem Gewinde versehen wird. Nach einem Gleitlochbohren bis zum Frakturspalt und dem Eindrehen der Schraube mit einem hierfür vorgesehenen Schlüssel kann - falls dies erforderlich ist - der überstehende Schraubenkopf abgesägt werden, wobei der Kragen 28 dann als Fixationsfläche für das Knochenfragment dient.

Bei einem nicht dargestellten Ausführungsbeispiel der Erfindung ist als Fixationselement ein Nagel in Form eines runden Knochenstiftes vorgesehen, der an einem Ende zugespitzt ist und am entgegengesetzten Ende einen flachen Kopf aufweist. Ein solcher Nagel dient zur Fixation osteokondraler Fragmente sowie zur Fixation bei verschiedenen Frakturen von kleinen Knochenfragmenten.

Die in Fig. 2 dargestellte Fixationsschraube dient zur Osteosynthese beispielsweise bei einer Radiusköpfchenfraktur, bei Basisfrakturen des fünften Mittelfußknochens, bei Kahnbeinfrakturen, bei Innenknöchelfrakturen, bei begleitenden Minimalosteosynthesen, bei Verwendung eines Fixateur externe, bei Patellafrakturen, die nicht auf Zug beansprucht werden, bei Tuberculum majus Frakturen und anderen Indikationen.

Die erfindungsgemäßen Fixationselemente aus kortikalem Knochenmaterial weisen im Gegensatz zu Knochenkeramiken, bei denen organische Materialien sowie Collagen verbrannt sind, sowohl Minerale wie Collagene auf, was den Heilungsprozeß deutlich beschleunigt. Obwohl Fixationselemente aus kortikalem Knochen schwächer sind als metallische Implantate, sind sie für viele Frakturen ausreichend stabil zur Fragmentfixation. Erfindungsgemäß hat sich herausgestellt, daß derartige Materialien aufgrund der besseren Verträglichkeit deutliche Vorteile mit sich bringen, insbesondere da eine zweite Operation zum Entfernen von Fremdkörpern entfallen kann. Für die Herstellung und Konservierung des erfindungsgemäßen Knochenmaterials kann ein Verfahren Verwendung finden, wie es in der DE 29 06 650 C2 beschrieben ist, auf die hiermit ausdrücklich Bezug genommen wird.

### Bezugszeichenliste

- 10: Fixationsstift
- 12: Hauptkörper
- 14: Abschnitt
- 16: Übergang
- 18: konisches Ende
- 20: Fixationsschraube
- 22: Schraubenkopf
- 24: Schraubenkörper
- 26: Ansatzfläche
- 27: Whitworth-Gewinde
- 28: Kragen
- 30: Setzwerkzeug
- 32: Führung
- 34: Führung
- 36: Schiene

- d: Durchmesser

## Patentansprüche

1. Fixationselement zur Osteosynthese im menschlichen oder tierischen Körper, das aus kortikalem Knochenmaterial besteht, das konserviert und sterilisiert ist, wobei das Fixationselement als Stift (10) oder Nagel ausgebildet ist und wobei das vordere Ende (18) des Fixationselementes konisch angespitzt ist,
**dadurch gekennzeichnet,**
**daß** im Bereich des vorderen Endes ein Abschnitt (14) mit verringertem Querschnitt vorgesehen ist, wobei der Übergang (16) zu dem Abschnitt (14) konisch verläuft.

2. Fixationselement nach Anspruch 1,
**dadurch gekennzeichnet,**
**daß** der Übergang (16) unter einem Winkel von etwa 45° zur Längsachse konisch verläuft.

3. Fixationselement nach Anspruch 1 oder 2,
**dadurch gekennzeichnet,**
**daß** sich der Abschnitt (14) an das konisch gespitzte Ende (18) anschließt.

4. Fixationselement nach zumindest einem der vorstehenden Ansprüche,
**dadurch gekennzeichnet,**
**daß** der Querschnitt des Abschnittes (14) etwa 80 - 85 % des Querschnittes des übrigen Fixationselementes (12) beträgt, vorzugsweise zwischen etwa 3 und 5 mm².

5. Fixationselement nach zumindest einem der vorstehenden Ansprüche,
**dadurch gekennzeichnet,**
**daß** sich der Abschnitt (14) über etwa 10 - 20 % der Gesamtlänge des Fixationselementes (10) erstreckt, vorzugsweise über etwa 10 mm.

6. Fixationselement nach zumindest einem der vorstehenden Ansprüche,
**dadurch gekennzeichnet,**
**daß** dieses im Querschnitt kreisförmig ausgebildet ist.

## Claims

1. Fixation element for osteosynthesis in a human or animal body, which consists of cortical bone matter which is conserved and sterilised, with the fixation element being made as a pin (10) or as a nail, and with the forward end (18) of the fixation element being conically sharpened,
**characterised in that**
a section (14) with a reduced cross-section is provided in the region of the forward end, with the transition (16) to the section (14) extending conically.

2. Fixation element in accordance with claim 1, **characterised in that** the transition (16) extends conically at an angle of approximately 45° to the longitudinal axis.

3. Fixation element in accordance with claim 1 or claim 2, **characterised in that** the section (14) adjoins the conically sharpened end (18).

4. Fixation element in accordance with at least one of the preceding claims, **characterised in that** the cross-section of the section (14) amounts to approximately 80 - 85 % of the cross-section of the remaining fixation element (12), preferably between approximately 3 and 5 mm².

5. Fixation element in accordance with at least one of the preceding claims, **characterised in that** the section (14) extends over approximately 10 - 20 % of the total length of the fixation element (10), preferably over approximately 10 mm.

6. Fixation element in accordance with at least one of the preceding claims, **characterised in that** it is designed with a circular cross-section.

## Revendications

1. Élément de fixation pour une ostéosynthèse dans un corps humain ou animal, qui est formé d'une matière osseuse corticale qui est conservée et stérilisée, l'élément de fixation étant conçu sous forme de tige (10) ou d'aiguille et l'extrémité avant (18) de l'élément de fixation étant taillée en pointe conique, **caractérisé en ce que**, dans la zone de l'extrémité avant, il est prévu une partie (14) avec une plus petite section, la zone de transition (16) vers la partie (14) étant conique.

2. Élément de fixation selon la revendication 1, **caractérisé en ce que** la zone de transition (16) est conique en formant un angle de 45° environ par rapport à l'axe longitudinal.

3. Élément de fixation selon la revendication 1 ou 2, **caractérisé en ce que** la partie (14) est aboutée à l'extrémité (18) taillée en pointe conique.

4. Élément de fixation selon au moins une des revendications précédentes, **caractérisé en ce que** la section transversale de la partie (14) équivaut à environ 80 à 85% de la section transversale de l'élément de fixation restant, se situant avantageusement entre 3 et 5 mm² environ.

5. Élément de fixation selon au moins une des revendications précédentes, **caractérisé en ce que** la partie (14) s'étend sur environ 10 à 20% de la longueur totale de l'élément de fixation (10) de préférence sur environ 10 mm.

6. Élément de fixation selon au moins une des revendications précédentes, **caractérisé en ce que** la section transversale de celui-ci est de forme circulaire.
